# EUROPEAN PATENT APPLICATION

(11) **EP 2 487 179 A1**
(43) Date of publication of application: **15.08.2012**
(21) Application number: 10822153.2
(22) Date of filing: 05.10.2010
(51) Int. Cl.: C07F 9/58, B01J 31/24, C07C 67/38, C07C 69/54, C07F 15/00, C07B 61/00

(54) **METAL COMPLEX, PYRIDYLPHOSPHINE COMPOUND, AND METHOD FOR PRODUCING ALKYL METHACRYLATE**

(30) Priority: 08.10.2009 JP 2009234102
(71) Applicant: Sumitomo Chemical Co., Ltd, Chuo-ku, Tokyo 104-8260 (JP)
(72) Inventor: KAWAMURA, Masato, Niihama-shi Ehime (JP)
(74) Representative: Berryman, Natalia Grace
(86) International application number: PCT/JP2010/067813
(87) International publication number: WO 2011/043483

(57) **Abstract**

A metal complex having a pyridylphosphine compound represented by the formula (1) 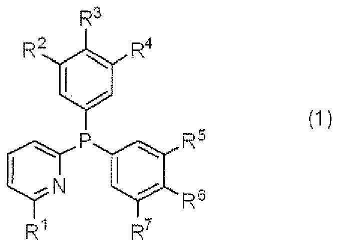 and a Group 10 metal atom of the periodic table.

## Description

### Technical Field

The present invention relates to a metal complex, a pyridylphosphine compound, and a method for producing an alkyl methacrylate.

### Background Art

With regard to metal complexes, a metal complex of which a center metal is palladium has been known. With regard to use of a palladium complex, a method for producing methyl methacrylate comprising reacting carbon monoxide, methanol and methylacetylene in the presence of a palladium complex having a 6-halo-2-pyridyldiphenylphosphine, and a protic acid, thereby producing methyl methacrylate in a production amount of 10,000 to 100,000 mole (methyl methacrylate)/mole (palladium atom).

### Disclosure of the Invention

The present invention relates to the following inventions.
[1] A metal complex having a pyridylphosphine compound represented by the formula (1) 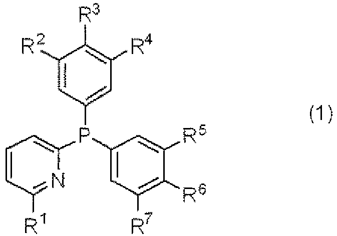 wherein R¹ represents a hydrogen atom, a halogen atom,
   a linear alkyl group having 1 to 20 carbon atom and optionally having a halogen atom as a substituent,
   an aralkyl group having 7 to 20 carbon atom and optionally having a halogen
   atom as a substituent,
   an aryl group having 6 to 20 carbon atom and optionally having a halogen atom as a substituent,
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent,
   R², R⁴, R⁵ and R⁷ independently each represent
   an alkyl group having 1 to 20 carbon atom and optionally having a halogen atom as a substituent,
   an aralkyl group having 7 to 20 carbon atom and optionally having a halogen atom as a substituent,
   an aryl group having 6 to 20 carbon atom and optionally having a halogen atom as a substituent,
   a silyl group represented by the following formula (2),
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent,
   R³ and R⁶ independently each represent a hydrogen atom, a halogen atom,
   an alkyl group having 1 to 20 carbon atom and optionally having a halogen atom as a substituent,
   an aralkyl group having 7 to 20 carbon atom and optionally having a halogen atom as a substituent,
   an aryl group having 6 to 20 carbon atom and optionally having a halogen atom as a substituent,
   a silyl group represented by the following formula (2),
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent, and
   two groups bonded to the neighboring carbon atoms among R², R³, R⁴, R⁵, R⁶ and R⁷ may be bonded each other to form a ring together with the carbon atoms to which they are bonded, and the formula (2) is represented by 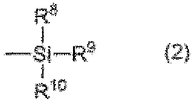 wherein R⁸, R⁹ and R¹⁰ independently each represent a hydrocarbon group having 1 to 20 carbon atoms,
   and a metal atom belonging to Group 10 of the periodic table.
[2] The metal complex according to [1], wherein the pyridylphosphine compound is a ligand.
[3] The metal complex according to [1] or [2], wherein R², R⁴, R⁵ and R⁷ independently each represent
   an alkyl group having 1 to 20 carbon atom and optionally having a halogen atom as a substituent,
   an aryl group having 6 to 20 carbon atom and optionally having a halogen atom as a substituent,
   a silyl group represented by the following formula (2),
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent.
[4] The metal complex according to any one of [1] to [3], wherein R¹ is a hydrogen atom, a halogen atom, a linear alkyl group having 1 to 4 carbon atoms, a benzyl group, a phenyl group, a methoxy group, a benzyloxy group or a phenoxy group, R², R⁴, R⁵ and R⁷ independently each is an alkyl group having 1 to 4 carbon atoms, an alkoxy group having 1 to 4 carbon atoms, a benzyl group, a phenyl group, a trimethylsilyl group, a tert-butyldimethylsilyl group or a phenoxy group, and R³ and R⁶ independently each is a hydrogen atom or an alkoxy group having 1 to 4 carbon atoms.
[5] The metal complex according to any one of [1] to [4], wherein R¹ is a hydrogen atom or a linear alkyl group having 1 to 4 carbon atoms, R², R⁴, R⁵ and R⁷ independently each is an alkyl group having 1 to 4 carbon atoms, an alkoxy group having 1 to 4 carbon atoms, a benzyl group, a phenyl group or a phenoxy group, and R³ and R⁶ independently each is a hydrogen atom or an alkoxy group having 1 to 4 carbon atoms.
[6] The metal complex according to any one of [1] to [5], wherein R¹ is a linear alkyl group having 1 to 4 carbon atoms, R², R⁴, R⁵ and R⁷ independently each is an alkyl group having 1 to 4 carbon atoms, and R³ and R⁶ independently each is a hydrogen atom, an alkyl group having 1 to 4 carbon atoms, or an alkoxy group having 1 to 4 carbon atoms.
[7] The metal complex according to any one of [1] to [6], wherein R¹ is a linear alkyl group having 1 to 4 carbon atoms, and R², R⁴, R⁵ and R⁷ are methyl groups.
[8] The metal complex according to any one of [1] to [5], wherein the pyridylphosphine compound is bis(3,5-dimethylphenyl)(6-methyl-2-pyridyl)phosphine, bis(3,5-dimethyl-4-methoxyphenyl)(6-methyl-2-pyridyl)phosphine, or bis(3,5-dimethylphenyl)(6-ethyl-2-pyridyl)phosphine.
[9] The metal complex according to any one of [1] to [5], wherein the pyridylphosphine compound is bis(3,5-dimethylphenyl)(6-methyl-2-pyridyl)phosphine.
[10] The metal complex according to any one of [1] to [5], wherein the pyridylphosphine compound is bis(3,5-dimethyl-4-methoxyphenyl)(6-methyl-2-pyridyl)phosphine.
[11] The metal complex according to any one of [1] to [5], wherein the pyridylphosphine compound is bis(3,5-dimethylphenyl)(6-ethyl-2-pyridyl)phosphine.
[12] The metal complex according to any one of [1] to [11], wherein the metal atom belonging to Group 10 of the periodic table is a palladium atom.
[13] A pyridylphosphine compound represented by the formula (1) 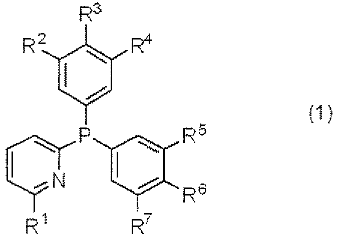 wherein R¹ represents a hydrogen atom, a halogen atom,
   a linear alkyl group having 1 to 20 carbon atom and optionally having a halogen atom as a substituent,
   an aralkyl group having 7 to 20 carbon atom and optionally having a halogen atom as a substituent,
   an aryl group having 6 to 20 carbon atom and optionally having a halogen atom as a substituent,
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent,
   R², R⁴, R⁵ and R⁷ independently each represent
   an alkyl group having 1 to 20 carbon atom and optionally having a halogen atom as a substituent,
   an aralkyl group having 7 to 20 carbon atom and optionally having a halogen atom as a substituent,
   an aryl group having 6 to 20 carbon atom and optionally having a halogen atom as a substituent,
   a silyl group represented by the following formula (2),
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent,
   R³ and R⁶ independently each represent a hydrogen atom, a halogen atom,
   an alkyl group having 1 to 20 carbon atom and optionally having a halogen atom as a substituent,
   an aralkyl group having 7 to 20 carbon atom and optionally having a halogen atom as a substituent,
   an aryl group having 6 to 20 carbon atom and optionally having a halogen atom as a substituent,
   a silyl group represented by the following formula (2),
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent, and
   two groups bonded to the neighboring carbon atoms among R², R³, R⁴, R⁵, R⁶ and R⁷ may be bonded each other to form a ring together with the carbon atoms to which they are bonded, and the formula (2) is represented by 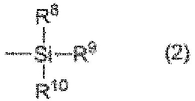 wherein R⁸, R⁹ and R¹⁰ independently each represent a hydrocarbon group having 1 to 20 carbon atoms.
[14] The pyridylphosphine compound according to [13], wherein R², R⁴, R⁵ and R⁷ independently each represent
   an alkyl group having 1 to 20 carbon atom and optionally having a halogen atom as a substituent,
   an aryl group having 6 to 20 carbon atom and optionally having a halogen atom as a substituent,
   a silyl group represented by the formula (2),
   an alkoxy group having 1 to 20 carbon atom and optionally having a halogen atom as a substituent,
   an aralkyloxy group having 7 to 20 carbon atom and optionally having a halogen atom as a substituent or
   an aryloxy group having 6 to 20 carbon atom and optionally having a halogen atom as a substituent.
[15] The pyridylphosphine compound according to [13], wherein R¹ is a hydrogen atom, a halogen atom, a linear alkyl group having 1 to 4 carbon atoms, a benzyl group, a phenyl group, a methoxy group, a benzyloxy group or a phenoxy group, R², R⁴, R⁵ and R⁷ independently each is an alkyl group having 1 to 4 carbon atoms, an alkoxy group having 1 to 4 carbon atoms, a benzyl group, a phenyl group, a trimethylsilyl group, a tert-butyldimethylsilyl group or a phenoxy group, and R³ and R⁶ independently each is a hydrogen atom or an alkyl group having 1 to 4 carbon atoms.
[16] The pyridylphosphine compound according to [13], wherein R¹ is a hydrogen atom or a linear alkyl group having 1 to 4 carbon atoms, R², R⁴, R⁵ and R⁷ independently each is an alkyl group having 1 to 4 carbon atoms, an alkoxy group having 1 to 4 carbon atoms, a benzyl group, a phenyl group or a phenoxy group, and R³ and R⁶ independently each is a hydrogen atom or an alkyl group having 1 to 4 carbon atoms.
[17] The pyridylphosphine compound according to any one of [13] to [16], wherein R¹ is a linear alkyl group having 1 to 4 carbon atoms, R², R⁴, R⁵ and R⁷ independently each is an alkyl group having 1 to 4 carbon atoms, and R³ and R⁶ independently each is a hydrogen atom, an alkyl group having 1 to 4 carbon atoms, or an alkoxy group having 1 to 4 carbon atoms.
[18] The pyridylphosphine compound according to any one of [13] or [14], which is bis(3,5-dimethylphenyl)(6-methyl-2-pyridyl)phosphine, bis(3,5-dimethyl-4-methoxyphenyl)(6-methyl-2-pyridyl)phosphine, or bis(3,5-dimethylphenyl)(6-ethyl-2-pyridyl)phosphine.
[19] A method for producing the pyridylphosphine compound according to any one of the above-mentioned [13] to [18], which comprises a first step of reacting a halogen-substituted pyridine compound represented by the formula (3) 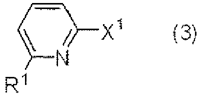 wherein R¹ is the same meaning as defined in the above-mentioned [13], and X¹ represents a halogen atom, with an alkyllithium compound, and
   a second step of reacting the reaction mixture obtained in the first step with a phosphine halide represented by the formula (4) 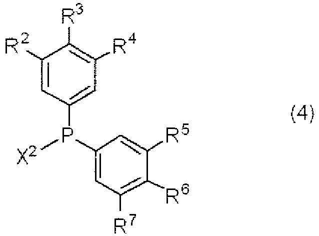 wherein R², R³, R⁴, R⁵, R⁶ and R⁷ are the same meaning as defined in the above-mentioned [13], and X² represents a halogen atom.
[20] A metal complex obtained by contacting the pyridylphosphine compound according to the above-mentioned [13] to [18] with a compound having a metal atom belonging to Group 10 of the periodic table.
[21] A method for producing an alkyl methacrylate comprising a step of reacting an acetylene compound, carbon monoxide and an alcohol in the presence of the metal complex according to any one of the above-mentioned [1] to [12] and a protic acid.

### Modes for Carrying Out the Invention

The present invention will be illustrated in detail below.

### <Pyridylphosphine Compound>

First, the pyridylphosphine compound of the present invention will be illustrated. The pyridylphosphine compound is represented by the formula (1) 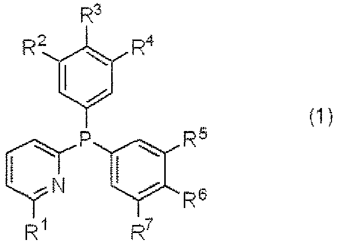 wherein R¹ represents a hydrogen atom, a halogen atom,
a linear alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent,
R², R⁹, R⁵ and R⁷ independently each represent
an alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
a silyl group represented by the following formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent,
R³ and R⁶ independently each represent a hydrogen atom, a halogen atom,
an alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
a silyl group represented by the following formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent, and
two groups bonded to the neighboring carbon atoms among R², R³, R⁴, R⁵, R⁶ and R⁷ may be bonded each other to form a ring together with the carbon atoms to which they are bonded, and the formula (2) is represented by 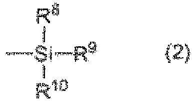 wherein R⁸, R⁹ and R¹⁰ independently each represent a hydrocarbon group having 1 to 20 carbon atoms.

In the present specification, a halogen atom may be any of a fluorine atom, a chlorine atom, a bromine atom and an iodine atom. Among these, preferred are a fluorine atom, a chlorine atom and a bromine atom as the halogen atom of R¹.

In the present specification, examples of the linear alkyl group having 1 to 20 carbon atom and optionally having a halogen atom as a substituent include a linear alkyl group having 1 to 20 carbon atom such as a methyl group, an ethyl group, a n-propyl group, a n-butyl group, a n-hexyl group, a n-octyl group, a n-decyl group, a n-dodecyl group, a n-hexadecyl group, a n-octadecyl group and a n-icosyl group, and a linear haloalkyl group having 1 to 20 carbon atom wherein one or more hydrogen atoms of the alkyl group illustrated here are replaced by a halogen atom. Also, the halogen atom may be any of those described above.

Among these, preferred is a linear alkyl group having 1 to 4 carbon atoms, and more preferred are a methyl group and an ethyl group.

In the present specification, examples of the aralkyl group having 7 to 20 carbon atom and optionally having a halogen atom as a substituent include an aralkyl group such as a benzyl group, a (2-methylphenyl)methyl group, a (3-methylphenyl)methyl group, a (4-methylphenyl)methyl group, a (2,3-dimethylphenyl)methyl group, a (2,4-dimethylphenyl)methyl group, a (2,5-dimethylphenyl)methyl group, a (2,6-dimethylphenyl)methyl group, a (3,4-dimethylphenyl)methyl group, a (3,5-dimethylphenyl)methyl group, a (2,3,4-trimethylphenyl)methyl group, a (2,3,5-trimethylphenyl)methyl group, a (2,3,6-trimethylphenyl)methyl group, a (3,4,5-trimethylphenyl)methyl group, a (2,4,5-trimethylphenyl)methyl group, a (2,4,6-trimethylphenyl)methyl group, a (2,3,4,5-tetramethylphenyl)methyl group, a (2,3,4,6-tetramethylphenyl)methyl group, a (2,3,5,6-tetramethylphenyl)methyl group, a (pentamethylphenyl)methyl group, a (4-ethylphenyl)methyl group, a [4-(n-propyl)phenyl]methyl group, a (4-isopropylphenyl)methyl group, a [4-(n-butyl)phenyl]methyl group, a [4-(sec-butyl)phenyl]methyl group, a [4-(tert-butyl)phenyl]methyl group, a [4-(n-pentyl)phenyl]methyl group, a (4-neopentylphenyl)methyl group, a [4-(n-hexyl)phenyl]methyl group, a [4-(n-octyl)phenyl]methyl group, a [4-(n-decyl)phenyl]methyl group, a [4-(n-dodecyl)phenyl]methyl group and a naphthylmethyl group, and a haloaralkyl group wherein one or more hydrogen atoms of the aralkyl group illustrated here are replaced by a halogen atom. Also, the halogen atom may be any of those described above. Among these, preferred is a benzyl group.

In the present specification, examples of the aryl group having 6 to 20 carbon atom and optionally having a halogen atom as a substituent include an aryl group such as a phenyl group, a 3-methylphenyl group, a 4-methylphenyl group, a 3,4-dimethylphenyl group, a 3,5-dimethylphenyl group, a 3,4,5-trimethylphenyl group, a 4-ethylphenyl group, a 4-(n-propyl)phenyl group, a 4-isopropylphenyl group, a 4-(n-butyl)phenyl group, a 4-(sec-butyl)phenyl group, a 4-(tert-butyl)phenyl group, a 4-(n-pentyl)phenyl group, a 4-(neopentyl)phenyl group, a 4-(n-hexyl)phenyl group, a 4-(n-octyl)phenyl group, a 4-(n-decyl)phenyl group, a 4-(n-dodecyl)phenyl group, a 4-(n-tetradecyl)phenyl group, a naphthyl group and an anthracenyl group, and a haloaryl group wherein one or more hydrogen atoms of the aryl group illustrated here are replaced by a halogen atom. In addition, the halogen atom may be any of those described above. Among these, preferred is a phenyl group.

In the present specification, examples of the alkoxy group having 1 to 20 carbon atom and optionally having a halogen atom as a substituent include an alkoxy group having 1 to 20 carbon atoms such as a methoxy group, an ethoxy group, a n-propoxy group, an isopropoxy group, a n-butoxy group, a sec-butoxy group, a tert-butoxy group, a n-pentyloxy group, a neopentyloxy group, a n-hexyloxy group, a n-octyloxy group, a nonyloxy group, a n-decyloxy group, a n-undecyloxy group, a n-dodecyloxy group, a n-tridecyloxy group, a n-tetradecyloxy group, a n-pentadecyloxy group, a n-hexadecyloxy group, a n-heptadecyloxy group, a n-octadecyloxy group, a n-nonadecyloxy group and a n-icosyloxy group, and a haloalkoxy group having 1 to 20 carbon atoms wherein one or more hydrogen atoms of the alkoxy group illustrated here are replaced by a halogen atom. Also, the halogen atom may be any of those described above. Among these, preferred is an alkoxy group having 1 to 4 carbon atoms, and more preferred is a methoxy group.

In the present specification, examples of the aralkyloxy group having 7 to 20 carbon atom and optionally having a halogen atom as a substituent include a group wherein an oxygen atom is bonded to a methylene part of the aralkyl group having 7 to 20 carbon atom and optionally having a halogen atom as a substituent illustrated above. Among them, more preferred is a benzyloxy group.

In the present specification, examples of the aryloxy group having 6 to 20 carbon atom and optionally having a halogen atom as a substituent include a group wherein an oxygen atom is bonded to the aryl group optionally having a halogen atom as a substituent illustrated above. Among them, more preferred is a phenoxy group.

In the present specification, examples of the alkyl group having 1 to 20 carbon atom and optionally having a halogen atom as a substituent also include a branched alkyl group such as an isopropyl group, a sec-butyl group, a tert-butyl group, a neopentyl group and an isopentyl group in addition to the linear alkyl group optionally having a halogen atom as a substituent. One or more hydrogen atoms in these branched alkyl groups may be replaced by a halogen atom, and examples of the halogen atoms include the those described above.

The inventor of the present invention has found that a metal complex capable of being used as a catalyst which produces the product in good yield can be obtained from the pyridylphosphine compound (1) wherein the atom bonded to the pyridine ring has no branched structure in R¹.

Therefore, the alkyl group of R¹ is needed to be linear, and the carbon number thereof is preferably 4 or less, and more preferably 2 or less.

When R¹ is the above-mentioned alkoxy, aralkyloxy and aryloxy groups, the atom bonded to the pyridine ring is an oxygen atom, and therefore, the part bonded to the pyridine ring has no branched structure. It is preferred that the bulk of each of the alkoxy, aralkyloxy and aryloxy groups is smaller, and therefore, the carbon number thereof is preferably smaller. The carbon number of the alkoxy group is preferably 4 or less, and more preferably 2 or less. The carbon number of the above-mentioned aralkyloxy group is preferably 10 or less, and more preferably 7. The carbon number of the above-mentioned aryloxy group is preferably 9 or less, and more preferably 6. The carbon number of the aryl group is preferably 9 or less, and more preferably 6.

It is preferred that R², R⁴, R⁵ and R⁷ independently each represent an alkyl group having 1 to 20 carbon atom and optionally having a halogen atom as a substituent, an aryl group having 6 to 20 carbon atom and optionally having a halogen atom as a substituent, the silyl group represented by the formula (2) described below, an alkoxy group having 1 to 20 carbon atom and optionally having a halogen atom as a substituent, an aralkyloxy group having 7 to 20 carbon atom and optionally having a halogen atom as a substituent or an aryloxy group having 6 to 20 carbon atom and optionally having a halogen atom as a substituent.

In R², R³, R⁴, R⁵, R⁶ and R⁷, the carbon number of the alkyl group is preferably 4 or less, and more preferably 2 or less.

In R², R³, R⁴, R⁵, R⁶ and R⁷, the carbon number of the aralkyl, aryl, alkoxy, aralkyloxy and aryloxy groups is preferably 18 or less from the viewpoint of easier production of the pyridylphosphine compound (1), and more preferably 9 or less.

In the formula (1), two groups bonded to the neighboring carbon atoms among R², R³, R⁴, R⁵, R⁶ and R⁷ may be bonded each other to form a ring together with the carbon atoms to which they are bonded. Specifically, example in which R² and R³, R³ and R⁴, R⁵ and R⁶, or R⁶ and R⁷ are bonded each other to form a condensed ring together with the pyridine ring or benzene ring to which they are bonded is shown. Examples of the ring formed by these neighboring groups include a saturated or an unsaturated hydrocarbon ring, and specific examples thereof include a cyclopropane ring, a cyclobutane ring, a cyclopentane ring, a cyclopentene ring, a cyclohexane ring, a cyclohexene ring, a cycloheptane ring, a cyclooctane ring, a benzene ring, a naphthalene ring and an anthracene ring. One or more hydrogen atoms being on these rings may be replaced by a halogen atom such as a chlorine atom and a fluorine atom, or the like.

In the present specification, examples of the hydrocarbon group include the above-mentioned alkyl group having 1 to 20 carbon atoms and the above-mentioned aryl group having 6 to 20 carbon atoms.

In the present specification, examples of the silyl group represented by the formula (2) include a silyl group wherein R⁸, R⁹ and R¹⁰ independently each is an alkyl group having 1 to 20 carbon atoms or an aryl group having 6 to 20 carbon atoms. Specific examples of the silyl group represented by the formula (2) include a trimethylsilyl group, a triethylsilyl group, a tripropylsilyl group, a tributylsilyl group and a tert-butyldimethylsilyl group.

Specific examples of the pyridylphosphine compound (1), preferable combinations of R¹, R², R³, R⁴, R⁵, R⁶ and R⁷ in the formula (1), include those shown in Table 1, Table 2, Table 3, Table 4 and Table 5 (hereinafter, referred to as "Table 1 to table 5").

In addition, each of symbols means the following; H: hydrogen atom, Me: methyl group, Et: ethyl group, n-Pr: n-propyl group, i-Pr: isopropyl group, n-Bu: n-butyl group, i-Bu: isobutyl group, t-Bu: t-butyl group, Bn: benzyl group, Ph: phenyl group, TMS: trimethylsilyl group, TBDMS: tert-butyldimethylsilyl group.

**Table 1**

| Compound | R¹ | R² | R³ | R⁴ | R⁵ | R⁶ | R⁷ |
|---|---|---|---|---|---|---|---|
| 1 | H | Me | H | Me | Me | H | Me |
| 2 | H | Et | H | Et | Et | H | Et |
| 3 | H | n-Pr | H | n-Pr | n-Pr | H | n-Pr |
| 4 | H | i-Pr | H | i-Pr | i-Pr | H | i-Pr |
| 5 | H | n-Bu | H | n-Pr | n-Pr | H | n-Pr |
| 6 | H | i-Bu | H | i-Bu | i-Pr | H | i-Bu |
| 7 | H | t-Bu | H | t-Bu | t-Bu | H | t-Bu |
| 8 | H | Bn | H | Bn | Bn | H | Bn |
| 9 | H | Ph | H | Ph | Ph | H | Ph |
| 10 | H | TMS | H | TMS | TMS | H | TMS |
| 11 | H | TBDMS | H | TBDMS | TBDMS | H | TBDMS |
| 12 | H | OMe | H | OMe | OMe | H | OMe |
| 13 | H | OPh | H | OPh | OPh | H | OPh |
| 14 | H | Me | OMe | Me | Me | OMe | Me |
| 15 | H | Et | OMe | Et | Et | OMe | Et |
| 16 | H | n-Pr | OMe | n-Pr | n-Pr | OMe | n-Pr |
| 17 | H | i-Pr | OMe | i-Pr | i-Pr | OMe | i-Pr |
| 18 | H | n-Bu | OMe | n-Pr | n-Pr | OMe | n-Pr |
| 19 | H | i-Bu | OMe | i-Bu | i-Pr | OMe | i-Bu |
| 20 | H | t-Bu | OMe | t-Bu | t-Bu | OMe | t-Bu |
| 21 | H | Bn | OMe | Bn | Bn | OMe | Bn |
| 22 | H | Ph | OMe | Ph | Ph | OMe | Ph |
| 23 | H | TMS | OMe | TMS | TMS | OMe | TMS |
| 24 | H | TBDMS | OMe | TBDMS | TBDMS | OMe | TBDMS |
| 25 | H | OMe | OMe | OMe | OMe | OMe | OMe |

**Table 2**

| Compound | R¹ | R² | R³ | R⁴ | R⁵ | R⁶ | R⁷ |
|---|---|---|---|---|---|---|---|
| 26 | Me | Me | H | Me | Me | H | Me |
| 27 | Me | Et | H | Et | Et | H | Et |
| 28 | Me | n-Pr | H | n-Pr | n-Pr | H | n-Pr |
| 29 | Me | i-Pr | H | i-Pr | i-Pr | H | i-Pr |
| 30 | Me | n-Bu | H | n-Pr | n-Pr | H | n-Pr |
| 31 | Me | i-Bu | H | i-Bu | i-Pr | H | i-Bu |
| 32 | Me | t-Bu | H | t-Bu | t-Bu | H | t-Bu |
| 33 | Me | Bn | H | Bn | Bn | H | Bn |
| 34 | Me | Ph | H | Ph | Ph | H | Ph |
| 35 | Me | TMS | H | TMS | TMS | H | TMS |
| 36 | Me | TBDMS | H | TBDMS | TBDMS | H | TBDMS |
| 37 | Me | OMe | H | OMe | OMe | H | OMe |
| 38 | Me | OPh | H | OPh | OPh | H | OPh |
| 39 | Me | Me | OMe | Me | Me | OMe | Me |
| 40 | Me | Et | OMe | Et | Et | OMe | Et |
| 41 | Me | n-Pr | OMe | n-Pr | n-Pr | OMe | n-Pr |
| 42 | Me | i-Pr | OMe | i-Pr | i-Pr | OMe | i-Pr |
| 43 | Me | n-Bu | OMe | n-Pr | n-Pr | OMe | n-Pr |
| 44 | Me | i-Bu | OMe | i-Bu | i-Pr | OMe | i-Bu |
| 45 | Me | t-Bu | OMe | t-Bu | t-Bu | OMe | t-Bu |
| 46 | Me | Bn | OMe | Bn | Bn | OMe | Bn |
| 47 | Me | Ph | OMe | Ph | Ph | OMe | Ph |
| 48 | Me | TMS | OMe | TMS | TMS | OMe | TMS |
| 49 | Me | TBDMS | OMe | TBDMS | TBDMS | OMe | TBDMS |
| 50 | Me | OMe | OMe | OMe | OMe | OMe | OMe |

**Table 3**

| Compound | R¹ | R² | R³ | R⁴ | R⁵ | R⁶ | R⁷ |
|---|---|---|---|---|---|---|---|
| 51 | Et | Me | H | Me | Me | H | Me |
| 52 | Et | t-Bu | H | t-Bu | t-Bu | H | t-Bu |
| 53 | Et | TMS | H | TMS | TMS | H | TMS |
| 54 | Et | OMe | H | OMe | OMe | H | OMe |
| 55 | Et | Me | OMe | Me | Me | OMe | Me |
| 56 | Et | t-Bu | OMe | t-Bu | t-Bu | OMe | t-Bu |
| 57 | Et | TMS | OMe | TMS | TMS | OMe | TMS |
| 58 | n-Pr | Me | H | Me | Me | H | Me |
| 59 | n-Pr | t-Bu | H | t-Bu | t-Bu | H | t-Bu |
| 60 | n-Pr | TMS | H | TMS | TMS | H | TMS |
| 61 | n-Pr | OMe | H | OMe | OMe | H | OMe |
| 62 | n-Pr | Me | OMe | Me | Me | OMe | Me |
| 63 | n-Pr | t-Bu | OMe | t-Bu | t-Bu | OMe | t-Bu |
| 64 | N-Pr | TMS | OMe | TMS | TMS | OMe | TMS |
| 65 | Bn | Me | H | Me | Me | H | Me |
| 66 | Bn | t-Bu | H | t-Bu | t-Bu | H | t-Bu |
| 67 | Bn | TMS | H | TMS | TMS | H | TMS |
| 68 | Bn | OMe | H | OMe | OMe | H | OMe |
| 69 | Bn | Me | OMe | Me | Me | OMe | Me |
| 70 | Bn | t-Bu | OMe | t-Bu | t-Bu | OMe | t-Bu |
| 71 | Bn | TMS | OMe | TMS | TMS | OMe | TMS |
| 72 | Ph | Me | H | Me | Me | H | Me |
| 73 | Ph | t-Bu | H | t-Bu | t-Bu | H | t-Bu |
| 74 | Ph | TMS | H | TMS | TMS | H | TMS |
| 75 | Ph | OMe | H | OMe | OMe | H | OMe |

**Table 4**

| Compound | R¹ | R² | R³ | R⁴ | R⁵ | R⁶ | R⁷ |
|---|---|---|---|---|---|---|---|
| 76 | Ph | Me | OMe | Me | Me | OMe | Me |
| 77 | Ph | t-Bu | OMe | t-Bu | t-Bu | OMe | t-Bu |
| 78 | Ph | TMS | OMe | TMS | TMS | OMe | TMS |
| 79 | OMe | Me | H | Me | Me | H | Me |
| 80 | OMe | t-Bu | H | t-Bu | t-Bu | H | t-Bu |
| 81 | OMe | TMS | H | TMS | TMS | H | TMS |
| 82 | OMe | OMe | H | OMe | OMe | H | OMe |
| 83 | OMe | Me | OMe | Me | Me | OMe | Me |
| 84 | OMe | t-Bu | OMe | t-Bu | t-Bu | OMe | t-Bu |
| 85 | OMe | TMS | OMe | TMS | TMS | OMe | TMS |
| 86 | OBn | Me | H | Me | Me | H | Me |
| 87 | OBn | t-Bu | H | t-Bu | t-Bu | H | t-Bu |
| 88 | OBn | TMS | H | TMS | TMS | H | TMS |
| 89 | OBn | OMe | H | OMe | OMe | H | OMe |
| 90 | OBn | Me | OMe | Me | Me | OMe | Me |
| 91 | OBn | t-Bu | OMe | t-Bu | t-Bu | OMe | t-Bu |
| 92 | OBn | TMS | OMe | TMS | TMS | OMe | TMS |
| 93 | OPh | Me | H | Me | Me | H | Me |
| 94 | OPh | t-Bu | H | t-Bu | t-Bu | H | t-Bu |
| 95 | OPh | TMS | H | TMS | TMS | H | TMS |
| 96 | OPh | OMe | H | OMe | OMe | H | OMe |
| 97 | OPh | Me | OMe | Me | Me | OMe | Me |
| 98 | OPh | t-Bu | OMe | t-Bu | t-Bu | OMe | t-Bu |
| 99 | OPh | TMS | OMe | TMS | TMS | OMe | TMS |

**Table 5**

| Compound | R¹ | R² | R³ | R⁴ | R⁵ | R⁶ | R⁷ |
|---|---|---|---|---|---|---|---|
| 100 | F | Me | H | Me | Me | H | Me |
| 101 | F | t-Bu | H | t-Bu | t-Bu | H | t-Bu |
| 102 | F | TMS | H | TMS | TMS | H | TMS |
| 103 | F | OMe | H | OMe | OMe | H | OMe |
| 104 | F | Me | OMe | Me | Me | OMe | Me |
| 105 | F | t-Bu | OMe | t-Bu | t-Bu | OMe | t-Bu |
| 106 | F | TMS | OMe | TMS | TMS | OMe | TMS |
| 107 | Cl | Me | H | Me | Me | H | Me |
| 108 | Cl | t-Bu | H | t-Bu | t-Bu | H | t-Bu |
| 109 | Cl | TMS | H | TMS | TMS | H | TMS |
| 110 | Cl | OMe | H | OMe | OMe | H | OMe |
| 111 | Cl | Me | OMe | Me | Me | OMe | Me |
| 112 | Cl | t-Bu | OMe | t-Bu | t-Bu | OMe | t-Bu |
| 113 | Cl | TMS | OMe | TMS | TMS | OMe | TMS |
| 114 | Br | Me | H | Me | Me | H | Me |
| 115 | Br | t-Bu | H | t-Bu | t-Bu | H | t-Bu |
| 116 | Br | TMS | H | TMS | TMS | H | TMS |
| 117 | Br | OMe | H | OMe | OMe | H | OMe |
| 118 | Br | Me | OMe | Me | Me | OMe | Me |
| 119 | Br | t-Bu | OMe | t-Bu | t-Bu | OMe | t-Bu |
| 120 | Br | TMS | OMe | TMS | TMS | OMe | TMS |

Among the pyridylphosphine compound (1) illustrated in Table 1 to Table 5, preferred is a compound wherein R¹ is a hydrogen atom, a halogen atom, a linear alkyl group having 1 to 4 carbon atoms, a benzyl group, a phenyl group, a methoxy group, a benzyloxy group or a phenoxy group, R², R⁴, R⁵ and R⁷ independently each is an alkyl group having 1 to 4 carbon atoms, an alkoxy group having 1 to 4 carbon atoms, a benzyl group, a phenyl group, a trimethylsilyl group, a tert-butyldimethylsilyl group or a phenoxy group, and R³ and R⁶ independently is a hydrogen atom or an alkoxy group having 1 to 4 carbon atom in the formula (1), and
more preferred is a compound wherein R¹ is a hydrogen atom or a linear alkyl group having 1 to 4 carbon atoms, R², R⁴, R⁵ and R⁷ independently each is an alkyl group having 1 to 4 carbon atoms, an alkoxy group having 1 to 4 carbon atoms, a benzyl group, a phenyl group or a phenoxy group, and R³ and R⁶ independently each is a hydrogen atom or an alkoxy group having 1 to 4 carbon atom in the formula (1),
still more preferred is a compound wherein R¹ is a hydrogen atom or a linear alkyl group having 1 to 4 carbon atoms, R², R⁴, R⁵ and R⁷ independently each is an alkyl group having 1 to 4 carbon atoms, and R³ and R⁶ independently each is a hydrogen atom, an alkyl group having 1 to 4 carbon atoms or an alkoxy group having 1 to 4 carbon atoms in the formula (1), and
especially preferred is a compound wherein R¹ is a hydrogen atom or a linear alkyl group having 1 to 4 carbon atoms, R², R⁴, R⁵ and R⁷ independently each is an alkyl group having 1 to 4 carbon atoms, and R³ and R⁶ independently each is a hydrogen atom or an alkoxy group having 1 to 4 carbon atoms in the formula (1). Also, especially preferable examples thereof include a compound wherein R¹ is a linear alkyl group having 1 to 4 carbon atoms, and R², R⁴, R⁵ and R⁷ are methyl groups in the formula (1).

Specific examples of the pyridylphosphine compound (1) include bis(3,5-dimethylphenyl)(2-pyridyl)phosphine [compound <1>], bis(3,5-dimethylphenyl)(6-methyl-2-pyridyl)phosphine [compound <26>], bis(3,5-dimethylphenyl)(6-ethyl-2-pyridyl)phosphine [compound <51>], bis(3,5-dimethyl-4-methoxyphenyl)(2-pyridyl)phosphine [compound <14>], bis(3,5-dimethyl-4-methoxyphenyl)(6-methyl-2-pyridyl)phosphine [compound <39>] and bis(3,5-dimethyl-4-methoxyphenyl)(6-ethyl-2-pyridyl)phosphine [compound <55>], and preferred are bis(3,5-dimethylphenyl)(6-methyl-2-pyridyl)phosphine [compound <26>], bis(3,5-dimethyl-4-methoxyphenyl)(6-methyl-2-pyridyl)phosphine [compound <39>] and bis(3,5-dimethyl-4-methoxyphenyl)(6-ethyl-2-pyridyl)phosphine [compound <55>], and especially preferred are bis (3,5-dimethylphenyl) (6-methyl-2-pyridyl) phosphine [compounds <26>] and bis (3,5-dimethyl-4-methoxyphenyl) (6-methyl-2-pyrdyl) phosphine [compound <39>.

Herein, the numbers in < > of [compound < >] described after the names of the pyridylphosphine compound (1) mean the numbers of the compounds described in Table 1 to Table 5.

### <Method for producing the pyridylphosphine compound (1) >

Next, the method for producing the pyridylphosphine compound (1) (hereinafter, sometimes referred to as the method of the present invention) will be illustrated.

The pyridylphosphine compound (1) can be produced by the method comprising a first step of reacting a halogen-substituted pyridyl compound represented by the formula (3) 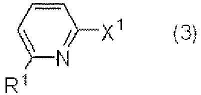 (hereinafter, sometimes referred to as "the halogen-substituted pyridyl compound (3)") with an alkyllithium compound, and a second step of reacting the reaction mixture obtained in the first step with a phosphine halide represented by the formula (4) 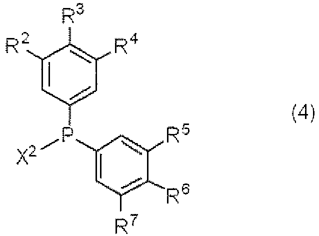 (hereinafter, sometimes referred to as "the phosphine halide compound (4) ") . This method will be illustrated in detail below.

In the above-mentioned formula (3), as X¹, a chlorine atom and a bromine atom are preferable. In the above-mentioned formula (3), R¹ and its preferable scope are the same as the definitions in the above-mentioned formula (1), and is especially preferably a linear alkyl group having 1 to 4 carbon atoms.

Specific examples of the halogen-substituted pyridyl compound (3) include 2-chloropyridine, 2-chloro-6-methylpyridine, 2-chloro-6-ethylpyridine, 2-chloro-6-n-propylpyridine, 2-chloro-6-isopropylpyridine, 2-chloro-6-n-butylpyridine, 2-bromopyridine, 2-bromo-6-methylpyridine, 2-bromo-6-ethylpyridine, 2-bromo-6-n-propylpyridine and 2-bromo-6-n-butylpyridine.

In the above-mentioned formula (4), as X², a chlorine atom and a bromine atom are preferable. In the above-mentioned formula (4), R², R³, R⁴, R⁵, R⁶ and R⁷ and their preferable scopes are the same as the definitions in the above-mentioned formula (1). It is especially preferred that in the above-mentioned formula (4), R², R⁴, R⁵ and R⁷ independently each is an alkyl group having 1 to 4 carbon atoms. R³ and R⁶ and their preferable scopes are the same as the definitions in the above-mentioned formula (1), and it is preferred that they independently each is a hydrogen atom, an alkyl group having 1 to 4 carbon atoms or an alkoxy group having 1 to 4 carbon atoms.

Examples of the combinations of X², R², R³, R⁴, R⁵, R⁶ and R⁷ in the formula (4) include those shown in Table 6 and Table 7. In addition, each of symbols in Table 6 and Table 7 are the same as those explained in Table 1 to table 5 described above.

**Table 6**

| Compound | X² | R_{'} | R³ | R⁴ | R⁵ | R⁶ | R⁷ |
|---|---|---|---|---|---|---|---|
| 201 | Cl | Me | H | Me | Me | H | Me |
| 202 | Cl | Et | H | Et | Et | H | Et |
| 203 | Cl | n-Pr | H | n-Pr | n-Pr | H | n-Pr |
| 204 | Cl | i-Pr | H | i-Pr | i-Pr | H | i-Pr |
| 205 | Cl | n-Bu | H | n-Pr | n-Pr | H | n-Pr |
| 206 | Cl | i-Bu | H | i-Bu | i-Pr | H | i-Bu |
| 207 | Cl | t-Bu | H | t-Bu | t-Bu | H | t-Bu |
| 208 | Cl | Bn | H | Bn | Bn | H | Bn |
| 209 | Cl | Ph | H | Ph | Ph | H | Ph |
| 210 | Cl | TMS | H | TMS | TMS | H | TMS |
| 211 | Cl | TBDMS | H | TBDMS | TBDMS | H | TBDMS |
| 212 | Cl | OMe | H | OMe | OMe | H | OMe |
| 213 | Cl | OPh | H | OPh | OPh | H | OPh |
| 214 | Cl | Me | OMe | Me | Me | OMe | Me |
| 215 | Cl | Et | OMe | Et | Et | OMe | Et |
| 216 | Cl | n-Pr | OMe | n-Pr | n-Pr | OMe | n-Pr |
| 217 | Cl | i-Pr | OMe | i-Pr | i-Pr | OMe | i-Pr |
| 218 | Cl | n-Bu | OMe | n-Pr | n-Pr | OMe | n-Pr |
| 219 | Cl | i-Bu | OMe | i-Bu | i-Pr | OMe | i-Bu |
| 220 | Cl | t-Bu | OMe | t-Bu | t-Bu | OMe | t-Bu |
| 221 | Cl | Bn | OMe | Bn | Bn | OMe | Bn |
| 222 | Cl | Ph | OMe | Ph | Ph | OMe | Ph |
| 223 | Cl | TMS | OMe | TMS | TMS | OMe | TMS |
| 224 | Cl | TBDMS | OMe | TBDMS | TBDMS | OMe | TBDMS |
| 225 | Cl | OMe | OMe | OMe | OMe | OMe | OMe |

**Table 7**

| Compound | X² | R² | R³ | R⁴ | R | R⁶ | R |
|---|---|---|---|---|---|---|---|
| 226 | Br | Me | H | Me | Me | H | Me |
| 227 | Br | Et | H | Et | Et | H | Et |
| 228 | Br | n-Pr | H | n-Pr | n-Pr | H | n-Pr |
| 229 | Br | i-Pr | H | i-Pr | i-Pr | H | i-Pr |
| 230 | Br | n-Bu | H | n-Pr | n-Pr | H | n-per |
| 231 | Br | i-Bu | H | i-Bu | i-Pr | H | i-Bu |
| 232 | Br | t-Bu | H | t-Bu | t-Bu | H | t-Bu |
| 233 | Br | Bn | H | Bn | Bn | H | Bn |
| 234 | Br | Ph | H | Ph | Ph | H | Ph |
| 235 | Br | TMS | H | TMS | TMS | H | TMS |
| 236 | Br | TBDMS | H | TBDMS | TBDMS | H | TBDMS |
| 237 | Br | OMe | H | OMe | OMe | H | OMe |
| 238 | Br | OPh | H | OPh | OPh | H | OPh |
| 239 | Br | Me | OMe | Me | Me | OMe | Me |
| 240 | Br | Et | OMe | Et | Et | OMe | Et |
| 241 | Br | n-Pr | OMe | n-Pr | n-Pr | OMe | n-Pr |
| 242 | Br | i-Pr | OMe | i-Pr | i-Pr | OMe | i-Pr |
| 243 | Br | n-Bu | OMe | n-Pr | n-Pr | OMe | n-Pr |
| 244 | Br | i-Bu | OMe | i-Bu | i-Pr | OMe | i-Bu |
| 245 | Br | t-Bu | OMe | t-Bu | t-Bu | OMe | t-Bu |
| 246 | Br | Bn | OMe | Bn | Bn | OMe | Bn |
| 247 | Br | Ph | OMe | Ph | Ph | OMe | Ph |
| 248 | Br | TMS | OMe | TMS | TMS | OMe | TMS |
| 249 | Br | TBDMS | OMe | TBDMS | TBDMS | OMe | TBDMS |
| 250 | Br | OMe | OMe | OMe | OMe | OMe | OMe |

As the phosphine halide compound (4), preferred is a compound wherein X² is a chlorine atom and a bromine atom, R², R⁴, R⁵ and R⁷ independently each is an alkyl group having 1 to 4 carbon atoms, an alkoxy group having 1 to 4 carbon atoms, a benzyl group, a phenyl group, a trimethylsilyl group, a tert-butyldimethylsilyl group or a phenoxy group, and R³ and R⁶ independently is a hydrogen atom or an alkoxy group having 1 to 4 carbon atom in the formula (4), and
more preferred is a compound wherein X² is a chlorine atom and a bromine atom, R², R⁴, R⁵ and R⁷ independently each is an alkyl group having 1 to 4 carbon atoms, an alkoxy group having 1 to 4 carbon atoms, a benzyl group, a phenyl group or a phenoxy group, and R³ and R⁶ independently each is a hydrogen atom or an alkoxy group having 1 to 4 carbon atoms in the formula (4),
still more preferred is a compounds wherein X² is a chlorine atom or a bromine atom, R², R⁴, R⁵ and R⁷ independently each is an alkyl group having 1 to 4 carbon atoms, and R³ and R⁶ independently each is a hydrogen atom, an alkyl group having 1 to 4 carbon atoms or an alkoxy group having 1 to 4 carbon atoms in the formula (4), and
especially preferred is a compound wherein X² is a chlorine atom or a bromine atom, R², R⁴, R⁵ and R⁷ independently each is an alkyl group having 1 to 4 carbon atoms, especially a methyl group, and R³ and R⁶ independently each is a hydrogen atom or an alkoxy group having 1 to 4 carbon atoms in the formula (4).

Specifically, preferable examples of the phosphine halide compound (4) include bis(3,5-dimethylphenyl)chlorophosphine [compound <201>], bis(3,5-dimephylphenyl)bromophosphine [compound <226>], bis (3, 5-dimethyl-4-methoxyphenyl) chlorophosphine [compound <214>] and bis(3,5-dimethyl-4-methoxyphenyl)bromophosphine [compound <239>], and more preferred are bis(3,5-dimethylphenyl)chlorophosphine [compound <201>], bis(3,5-dimethyl-4-methoxyphenyl)chlorophosphine [compound <214>] and bis(3,5-dimethylphenyl)chlorophosphine [compound <201>], and still more preferred are bis(3,5-dimethylphenyl)chlorophosphine [compound <201>] and bis(3,5-dimethyl-4-methoxyphenyl)chlorophosphine [compound <214>] .

Herein, the numbers in < > of [compound < >] described after the names of the phosphine halide compound (4) mean the numbers of the compounds described in Table 6 and Table 7.

In the method of the present invention, as the alkyllithium compound, an alkyllithium having an alkyl group having 1 to 5 carbon atoms is preferable. Specific examples of the alkyllithium compound include methyllithium, ethyllithium, propyllithium and butyllithium.

While a solvent is not necessarily needed in the method of the present invention, it is preferably conducted in the presence of a solvent.

As the solvent used in the method of the present invention, ether solvents such as tetrahydrofuran [THF] and diethyl ether, or hydrocarbon solvents such as hexane are preferable, and these may be alone, or may be a mixed solvent mixed with two or more kinds thereof.

In the above-mentioned method, the first step may be conducted by reacting the halogen-substituted pyridyl compound (3) with the alkyllithium compound in the presence of the solvent followed by conducting the second step by adding the phosphine halide (4) to the reaction mixture obtained in the first step, and the first step may be conducted by mixing the halogen-substituted pyridyl compound (3) with the alkyllithium compound followed by conducting the second step by adding the reaction mixture obtained in the first step to the phosphine halide (4). From the viewpoint of easy operation, the former is preferable.

Also, when the ratio of the amount to be used of the halogen-substituted pyridyl compound (3) to the alkyllithium compound (molar ratio) is expressed as [the halogen-substituted pyridyl compound (3)] / [he alkyllithium compound] in the method, it is preferably a range of 1/0.5 to 1/1.5, and more preferably a range of 1/0.75 to 1/1.3.

The ratio of the amount to be used of the halogen-substituted pyridyl compound (3) to the phosphine halide (4) (molar ratio) is preferably a range of 1/0.5 to 1/2.

The reaction conditions of the method of the present invention will be illustrated.

The reaction temperature in each step is selected from the range of -80°C to the boiling point of the solvent used or less, and preferably a range of -80°C to about 100°C. Also, the reaction temperature of the first step may be same as or different from that of the second step, and the reaction temperature of the first step is preferably lower that that of the second step.

When the reactions are carried out at the above-mentioned temperature range, the reaction temperatures are sufficient to be within 48 hours.

After the reaction, as necessary, the temperature of the reaction mixture is set at about room temperature, and then, the pyridylphosphine compound (1) which is a product can be obtained by operations such as extraction, recrystallization, reprecipitation, or various chromatography or a combination thereof.

An aqueous phase containing inorganic components generated in the production steps and an organic phase containing the pyridylphosphine compound (1) and the solvent can be separated by adding water or aqueous salt solution to the reaction mixture after the second step. Examples of the above-mentioned aqueous salt solution include aqueous sodium chloride solution, aqueous sodium sulfate solution, aqueous potassium solution and aqueous sodium hydrogen carbonate solution. Further, the pyridylphosphine compound (1) can be separated and purified by concentrating the organic layer obtained by the above-mentioned separation and then purifying the concentrate obtained with column chromatography or by conducting recrystallization of the organic layer. As necessary, the pyridylphosphine compound (1) can also be extracted by adding an organic solvent to the aqueous phase obtained in the above-mentioned separation.

### <Metal Complex>

The metal complex of the present invention has the pyridylphosphine compound represented by the formula (1) described above and a metal atom belonging to Group 10 of the periodic table. Hereinafter, "metal belonging to Group 10 of the periodic table" is sometimes referred to as "Group 10 metal".

In the metal complex of the present invention, the pyridylphosphine compound represented by the formula (1) is usually a ligand. Group 10 metal atom usually exists as a center atom of the metal complex.

Examples of Group 10 metal atom include nickel atom, palladium atom and platinum atom, and palladium atom is preferable.

The metal complex of the present invention can be obtained from the pyridylphosphine compound (1) and metal simple substance of Group 10 metal or a compound having Group 10 metal, preferably from the pyridylphosphine compound (1) and a compound having Group 10 metal.

The metal complex of the present invention is preferably obtained by contacting the pyridylphosphine compound (1) with the compound having Group 10 metal.

When Group 10 metal atom is palladium atom, examples of the compound having Group 10 metal for obtaining the metal complex of the present invention include palladium acetylacetonate, tetrakis(triphenylphosphine)palladium, bis(triphenylphosphine)palladium acetate, palladium chloride and palladium acetate.

In the preparation of the above-mentioned metal complex, the amount to be used of the above-mentioned metal simple substance or the above-mentioned metal compound and the pyridylphosphine compound (1) are not limited, and the amount to be used of the pyridylphosphine compound (1) is preferably a range of 3 to 400 moles relative to 1 mole of the above-mentioned metal simple substance or the above-mentioned metal compound, and more preferably a range of 10 to 240 moles.

When the metal complex of the present invention is prepared, at least one solvent selected from the group consisting of alcohol solvents, sulfur-containing solvents, hydrocarbon solvents, halogen-containing hydrocarbon solvents, ester solvents, ether solvents and amide solvents is preferably present therein.

Examples of the above-mentioned alcohol solvents include methanol, ethanol, 2-propanol, tert-butanol and ethylene glycol. Examples of the above-mentioned sulfur-containing solvents include dimethyl sulfoxide and sulfolane. Examples of the above-mentioned hydrocarbon solvents include hexane, toluene and xylene. Examples of the above-mentioned halogen-containing hydrocarbon solvents include dichloromethane, dichloroethane, trichloroethane, tetrachloroethane, chlorobenzene and dichlorobenzene. Examples of the above-mentioned ester solvents include methyl acetate, ethyl acetate and methyl methacrylate. Examples of the above-mentioned ether solvents include acetone, methyl ethyl ketone, methyl isobutyl ketone, anisole, dimethoxyethane, diglyme, methyl-tert-butyl ether, dibutyl ether and THF. Examples of the above-mentioned amide solvents include N,N-dimethylformamide, N-methylpyrrolidone and N,N-dimethylacetamide. When the above-mentioned metal complex is used for the alkoxycarbonylation reaction described below, the solvent used in the above-mentioned preparation is preferably same as alcohol used in the alkoxycarbonylation reaction.

The preparation temperature of the preparation of the metal complex of the present invention is preferably a range of 0 to 100°C. When the reaction is carried out at the temperature within the above-mentioned range, the above-mentioned metal simple substance or the above-mentioned metal compound is mixed with the pyridylphosphine compound (1) generally for 0.25 to 48 hours, thereby be able to contact these enough.

When the metal complex of the present invention is used for the alkoxycarbonylation reaction described below, the preparation of the metal complex may be conducted in the presence of the protonic acid and the amine compound described below. In such case, an alcohol preferably exists therein as a solvent, and the alcohol used as the reaction agent of the alkoxycarbonylation reaction especially preferably exists therein.

A metal complex solution containing the metal complex of the present invention can be obtained by the above-mentioned contact. The metal complex can be separated and purified from the metal complex solution with the known method such as recrystallization, concentration and column chromatography.

The metal complex of the present invention can be used for various reactions such as polymerization reaction, oligomerization reaction, alkylation reaction, Heck reaction, hydrogenation reaction, coupling reaction, oxidation reaction, carboxylation reaction, alkoxycarbonylation reaction, hydroformylation reaction and hydrosilylation reaction.

As the metal complex of the present invention, the metal complex separated from the above-mentioned metal complex solution by suitable operation may be used as the catalyst for various reactions, and the metal complex solution may be used as the catalyst for various reactions without separating the metal complex.

### <Alkoxycarbonylation Reaction>

The metal complex of the present invention is especially useful as the catalyst of the reaction wherein an acetylene compound is subjected to alkoxycarbonylation reaction to obtain an alkyl methacrylate. The alkoxycarbonylation reaction of the acetylene compound described here means a reaction wherein an alkyl methacrylate is produced by reacting an acetylene compound, carbon monoxide and an alcohol.

In the alkoxycarbonylation reaction, the metal complex separated and purified can be also used as the catalyst of the alkoxycarbonylation reaction, and the reaction can be also conducted by providing the acetylene compound, carbon monoxide and the alcohol to the metal complex solution obtained by contacting metal simple substrate of Group 10 metal or the compound having Group 10 metal with the pyridylphosphine compound (1).

The used amount ratio of the acetylene compound to the metal complex of the present invention is preferably set so that Group 10 metal atom can be in a range of 10⁻² to 10⁻⁶ mole per 1 mole of the acetylene compound. When the acetylene compound is provided to the metal complex not isolated, the amount of metal simple substrate of Group 10 metal or the compound having the metal may be set in a range of 10⁻² to 10⁻⁶ mole per 1 mole of the acetylene compound.

In the above-mentioned alkoxycarbonylation reaction, a protonic acid is usually used in combination in addition to the metal complex of the present invention. Examples of the protonic acid include phosphoric acids such as orthophosphoric acid, pyrophosphoric acid, polyphosphoric acid and benzenephosphoric acid; sulfuric acid; hydrohalic acids; sulfonic acids such as benzenesulfonic acid, p-toluenesulfonic acid, naphthalenesulfonic acid, chlorosulfonic acid, methanesulfonic acid, trifluoromethanesulfonic acid and trimethylmethanesulfonic acid; and carboxylic acids such as monochloroacetic acid, dichloroacetic acid, trichloroacetic acid, trifluoroacetic acid and oxalic acid. Two or more kinds thereof may be mixed to be used.

Among them, preferred is the sulfonic acid, and more preferred is methanesulfonic acid. While the used amount of the protonic acid is not limited, it is preferably in a range of 3 to 600 moles per 1 mole of Group 10 metal atom in the metal complex of the present invention, and more preferably in a range of 10 to 360 moles.

The acetylene compound in the above-mentioned alkoxycarbonylation reaction is a compound having a carbon-carbon triple bond, and its carbon number is preferably 2 to 20. Preferable examples of the acetylene compound include acetylene, methylacetylene, cyclohexylacetylene, phenylacetylene, 1-butyne, 1-pentyne, 1-hexane, 1-heptyne, 1-octyne, 2-butyne, 3-hexyne and diphenylacetylene.

Among them, methylacetylene is especially useful for the alkoxycarbonylation reaction in which industrially useful methyl methacrylate is obtained. If hydrocarbons such as allene, butane and propylene are contained as impurities in methylacetylene, the alkoxycarbonylation reaction can be progressed enough by using the metal complex of the present invention as the catalyst, and therefore, the metal complex of the present invention is especially effective for the alkoxycarbonylation reaction, and with regard to allene, however, it sometimes decreases the catalytic activity of the metal complex of the present invention, and therefore, the amount of allene is preferably 1 part by weight or less per 100 parts by weight of the acetylene compound, and more preferably 0.1 pat by weight or less, and still more preferably 0.005 part by weight or less.

The alcohol used in the above-mentioned alkoxycarbonylation reaction is not especially limited, and the alkyl methacrylate corresponding to the alcohol can be produced. Examples of the alcohol in the above-mentioned reaction include an aliphatic alcohol having 1 to 6 carbon atoms, and an aliphatic alcohol having 1 to 4 carbon atoms is preferable, and an alcohol selected from the group consisting of methanol, ethanol, 1-propanol, 2-propanol, 1-butanol, sec-butanol and ethylene glycol is still more preferable.

In addition, in order to obtain methyl methacrylate from methylacetylene, methanol may be used as the alcohol.

The used amount of the alcohol is usually 0.5 mole or more per 1 mole of the acetylene compound, and preferably 1 mole or more, and it may be usually 20 moles or less. The use of excess amount of the alcohol is still more preferably since the reaction can be carried out in the absence of a solvent along with being able to increase the yield.

The above-mentioned alkoxycarbonylation reaction can be conducted in the presence of a solvent.

Examples of the solvent in the above-mentioned alkoxycarbonylation reaction include sulfur-containing solvents such as dimethyl sulfoxide, diisopropyl sulfone and sulfolane; aromatic hydrocarbon solvents such as benzene, toluene and xylene; ester solvents such as methyl acetate, ethyl acetate and butyl acetate; ketone solvents such as acetone and methyl isobutyl ketone; ether solvents such as anisole, dimethoxyethane, diglyme, methyl-tert-butyl ether, ethyl-tert-butyl ether, dibutyl ether and diisopropyl ether; and amide solvents such as dimethylformamide, N-methylpyrrolidone and dimethylacetamide. These solvents may be a mixture of two or more kinds thereof. The used amount of the other solvent is not especially limited.

In the above-mentioned alkoxycarbonylation reaction, still more preferable reaction result is sometimes obtained by adding an amine compound in addition to the protonic acid.

As the amine compound, a tertiary amine or a cyclic amine is preferable. Specific examples thereof include N,N-dialkylaniline, pyridine, quinoline, isoquinoline, triazine, imidazole, triethylamine, tributylamine and N,N-diisopropylethylamine, and two or more kinds selected from these may be used, and N,N-dimethylaniline or pyridine is preferable.

When the amine compound is added, the addition amount thereof is not limited, and it is preferably in a range of 1 to 50 moles per 1 mole of the protonic acid, and more preferably in a range of 1 to 10 moles.

The alkoxycarbonylation reaction can be carried out by providing the acetylene compound and carbon monoxide to a mixture of the metal complex of the present invention, the alcohol and the protonic acid, and the solvent added if necessary, preferably to a mixture of the metal complex of the present invention, the alcohol, the protonic acid, the amine compound and the solvent.

While the mixing order of the metal complex of the present invention, the alcohol and the like is not limited in the above-mentioned alkoxycarbonylation reaction, it is preferred that the metal complex solution obtained by contacting metal simple substrate of Group 10 metal or the compound having Group 10 metal with the pyridylphosphine compound (1) is previously mixed with the alcohol, the protonic acid and the amine compound, and then, the acetylene compound is added to the mixed liquid obtained. Further, the reaction can be efficiently conducted by conducting the contact in the presence of the alcohol used in the reaction and mixing the metal complex solution obtained with the protonic acid and the amine compound.

Carbon monoxide used in the above-mentioned alkoxycarbonylation reaction is not necessarily pure carbon monoxide, and may contain gases significantly preventing the above-mentioned alkoxycarbonylation reaction from progressing such as nitrogen and argon. The partial pressure of carbon monoxide on the reaction is preferably 0.1 to 10 MPaG.

The reaction temperature of the above-mentioned alkoxycarbonylation reaction is preferably a range of 20 to 100°C, and more preferably a range of 50 to 90°C. The reaction time can be accordingly adjusted depending on the kind of the metal complex of the present invention, and the kind and the amount of the acetylene compound, the alcohol and the protonic acid, and it is preferably 12 to 24 hours. The reaction pressure is preferably a range of 0.5 to 10 MPaG and more preferably a range of 1 to 9 MPaG.

After the above-mentioned alkoxycarbonylation reaction, the alkyl methacrylate can be isolated and purified by a operation such as distillation, extraction, recrystallization and various chromatography or a combination thereof. For example, methyl methacrylate obtained by using methylacetylene as the acetylene compound and by using methanol as the alcohol can be purified by a operation such as distillation and/or extraction or the like.

Examples of the alkyl methacrylate obtained by the above-mentioned alkoxycarbonylation reaction include an alkyl methacrylate having an alkyl group having 1 to 4 carbon atoms. Specific examples of the alkyl methacrylate include methyl methacrylate, ethyl methacrylate, propyl methacrylate and butyl methacrylate.

### <Use of Alkyl Methacrylate>

The alkyl methacrylate thus obtained can be used as a raw material of various industrial materials. Among the alkyl methacrylates, illustrated is the use of methyl methacrylate.

Polymethylmethacrylate obtained from methyl methacrylate and a copolymer obtained from methyl methacrylate and the other olefin or the like can be applied for various uses such as building materials, electronic materials or separation materials. These polymethylmethacrylate and copolymer can be produced according to the known polymerization means (suspension polymerization process, bulk polymerization process, solution polymerization process or the like).

### Examples

The present invention will be illustrated by Examples in more detail below. Also, methylacetylene was used assuming that its purity was 99% unless otherwise specifically noted. Unless otherwise specifically noted, % is on a weight basis.

### Example 1

2-Bromo-6-methylpyridine (18.9 mmol) was dissolved in 40 ml of tetrahydrofuran [THF], and 1.59 M hexane solution of n-butyllithium (18.9 mmol) was added dropwise thereto with cooling with dry ice-ethanol bath and with stirring. Then, the mixed liquid obtained was stirred for 10 minutes to obtain a reaction mixture. To the reaction mixture obtained, chlorobis(3,5-dimethyl-4-methoxyphenyl)phosphine (17.5 mmol) dissolved in 20 ml of THF was added, and the temperature was brought back to room temperature and the stirring was carried out under room temperature for 2 hours to conduct the reaction. To the solution obtained, a little amount of water was added to quench the reaction, and a saturated aqueous sodium chloride solution was further added thereto. Next, to the solution obtained, ethyl acetate was added to stir and separate, and then, an ethyl acetate phase (an organic phase) was collected by separation. This organic phase was dried over anhydrous magnesium sulfate, and filtrated, and the filtrate obtained was concentrated under reduced pressure.

The concentrate obtained was purified with silica gel chromatography (developer: hexane/ethyl acetate =5/1, weight ratio) to obtain 4.45 g of bis(3,5-dimethyl-4-methoxyphenyl)(6-methyl-2-pyridyl)phosphine (Yield 81%).

From the result of analysis with ¹H-NMR (¹H-nuclear magnetic resonance spectrum), its purity was calculated it was 99%.

¹H-NMR (CDCl₃, 270 MHz) : δ 7.42 (1H, td, J=8.2 Hz), 6.99 (5H, pseudo d, J=8 Hz), 6.82 (1H, d, J=8 Hz), 3.71 (6H, s) 2.55 (3H, s), 2.22 (12H, s)

### Example 2

2-Bromo-6-methylpyridine (19.5 mmol) was dissolved in 40 ml of tetrahydrofuran [THF], and 1.59 M hexane solution of n-butyllithium (19.5 mmol) was added dropwise thereto with cooling with dry ice-ethanol bath and with stirring. Then, the mixed liquid obtained was stirred for 10 minutes to obtain a reaction mixture. To the reaction mixture obtained, chlorobis (3,5-dimethylphenyl)phosphine (18.8 mmol) dissolved in 20 ml of THF was added, and the temperature was brought back to room temperature and the stirring was carried out under room temperature for 2 hours to conduct the reaction. To the solution obtained, a little amount of water was added to quench the reaction, and a saturated aqueous sodium chloride solution was further added thereto. Next, to the solution obtained, ethyl acetate was added to stir and separate, and then, an ethyl acetate phase (an organic phase) was collected by separation. This organic layer was dried over anhydrous magnesium sulfate, and filtrated, and the filtrate obtained was concentrated under reduced pressure. The concentrate obtained was purified twice with silica gel chromatography (developer: hexane/ethyl acetate =5/1, 9/1, weight ratio) to obtain 3.68 g of bis(3,5-dimethylphenyl) (6-methyl-2-pyridyl)phosphine (Yield 59%). The purity calculated with ¹H-NMR analysis was 99%.

¹H-NMR (CDCl₃, 270 MHz) : δ 7.42 (1H, td, J=8.2 Hz), 7.25-7.03 (7H, m), 6.83 (1H, d, J=8 Hz), 2.56 (3H, s), 2.26 (12H, s)

### Example 3

2-Bromo-6-ethylpyridine (18.2 mmol) was dissolved in 40 ml of tetrahydrofuran [THF], and 1.65 M hexane solution of n-butyllithium (19.1 mmol) was added dropwise thereto with cooling with dry ice-ethanol bath and with stirring. Then, the mixed liquid obtained was stirred for 10 minutes to obtain a reaction mixture. To the reaction mixture obtained, chlorobis (3,5-dimethylphenyl) phosphide (16.8 mmol) dissolved in 20 ml of THF was added, and the temperature was brought back to room temperature and the stirring was carried out under room temperature for 3 hours to conduct the reaction. To the solution obtained, a little amount of water was added to quench the reaction, and a saturated aqueous sodium chloride solution was further added thereto. Next, to the solution obtained, ethyl acetate was added to stir and separate, and then, an ethyl acetate phase (an organic phase) was collected by separation. This organic layer was dried over anhydrous magnesium sulfate, and filtrated, and the filtrate obtained was concentrated under reduced pressure. The concentrate obtained was purified twice with silica gel chromatography (developer: hexane/ethyl acetate = 20/1) to obtain 2.07 g of bis(3,5-dimethylphenyl)(6-ethyl-2-pyridyl)phosphine (Yield 33%). The purity calculated with ¹H-NMR analysis was 97%.

¹H-NMR (CDCl₃, 600 MHz) : 5 7.44 (td, 1H, J=8.2 Hz), 7.03-6.95 (m, 7H), 7.03 (d, 1H, J=8 Hz), 6.83 (d, 1H, J=8 Hz), 2.83 (q, 2H, J=8 Hz), 2.26 (s, 12H), 1.27 (t, 3H, J= 8Hz)

### Example 4

A metal complex solution was obtained by mixing enough 45 mL of methanol with 1.8 mg (0.0080 mmol) of palladium acetate and 127 mg (0.32 mmol) of bis(3,5-dimethyl-4-methoxyphenyl) (6-methyl-2-pyridyl)phosphine obtained in Example 1. To the metal complex solution obtained, 0.10 ml (0.80 mmol) of N,N-dimethylaniline and 31 µl (0.48 mmol) of methanesulfonic acid were added to prepare a metal complex solution 1.

### Example 5

One point seven (1.7) ml of the metal complex solution 1 obtained in Example 4 (corresponding to 0.00030 mmol in terms of palladium atom) was taken, and introduced into a stainless-steel autoclave of which inner volume was 100 ml under an atmosphere of nitrogen, and then, 28.3 ml of methanol was further introduced into the autoclave. The autoclave was cooled with dry ice-ethanol bath, and 6.40 g (158 mmol) of methylacetylene in which propadiene concentration was 20 ppm by weight was introduced into this autoclave. Further, the inside of the autoclave was pressurized with carbon monoxide (CO) to adjust the pressure in the autoclave to 5 MPaG.

The maintaining at the reaction temperature of 65°C was conducted for 7 hours, and in order to keep the partial pressure of CO at 5 MPaG during this, carbon monoxide of which amount was that of carbon monoxide consumed was constantly introduced therein with a pressure reducing valve. The reaction mixture after the reaction was sampled, and the quantitative analysis thereof was performed with gas chromatography (GC) to find out that the yield of methyl methacrylate was 73% and the production amount of methyl methacrylate per 1 mole of palladium atom (Pd) existing in the palladium complex introduced was 380 thousand moles.

### Example 6

The metal complex solution 2 containing a palladium complex, N,N-dimethylaniline and methanesulfonic acid was prepared according to the same experiment as that of Example 4 except that 108 mg (0.32 mmol) of bis(3,5-dimethylphenyl) (6-methyl-2-pyridyl)phosphine was used in place of 127 mg of bis (3,5-dimethyl-4-methoxyphenyl) (6-methyl-2--pyridyl)phosphine.

### Example 7

The experiment was carried out according to the same experiment as that of Example 5 except that the metal complex solution 2 obtained in Example 6 was used in place of the metal complex solution 1, and 6.05 g (149 mmol) of methylacetylene in which propadiene concentration was 27 ppm by weight was used as methylacetylene. The production amount of methyl methacrylate per 1 mole of Pd existing in the palladium complex was 420 thousand moles.

### Example 8

The metal complex solution 3 containing a palladium complex, N,N-dimethylaniline and methanesulfonic acid was prepared according to the same experiment as that of Example 4 except that 115 mg (0.32 mmol) of bis (3,5-dimethylphenyl) (6-ethyl-2-pyridyl) phosphine was used in place of 127 mg of bis(3,5-dimethyl-4-methoxyphenyl) (6-methyl-2-pyridyl)phosphine.

### Example 9

The experiment was carried out according to the same experiment as that of Example 5 except that the metal complex solution 3 obtained in Example 8 was used in place of the metal complex solution 1, and 7.72 g (191 mmol) of methylacetylene in which propadiene concentration was 8.4 ppm by weight was used as methylacetylene. The production amount of methyl methacrylate per 1 mole of Pd existing in the palladium complex was 370 thousand moles.

### Comparative Example 1

The experiment was carried out according to the same experiment as that of Example 4 except that 112 mg (0.32 mg) of diphenyl(6-bromo-2-pyridyl)phosphine was used in place of 127 mg of bis (3, 5-dimethyl-4-methoxyphenyl) (6-methyl-2-pyridyl) phosphine, and 6.07 g (150 mmol) of methylacetylene in which propadiene concentration was 18 ppm by weight was used as methylacetylene. The production amount of methyl methacrylate per 1 mole of Pd existing in the palladium complex was mere 20 thousand moles.

### Industrial Applicability

The metal complex of the present invention is fairly useful for producing an alkyl methacrylate from an acetylene compound.

## Claims

1. A metal complex having a pyridylphosphine compound represented by the formula (1) 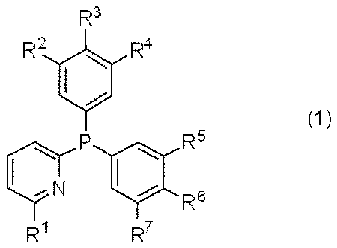 wherein R¹ represents a hydrogen atom, a halogen atom,
a linear alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent,
R², R⁴, R⁵ and R⁷ independently each represent
an alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
a silyl group represented by the following formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent,
R³ and R⁶ independently each represent a hydrogen atom, a halogen atom,
an alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
a silyl group represented by the following formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent, and
two groups bonded to the neighboring carbon atoms among R², R³, R⁴, R⁵, R⁶ and R⁷ may be bonded each other to form a ring together with the carbon atoms to which they are bonded, and the formula (2) is represented by 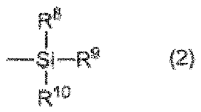 wherein R⁸, R⁹ and R¹⁰ independently each represent a hydrocarbon group having 1 to 20 carbon atoms,
and a metal atom belonging to Group 10 of the periodic table.

2. The metal complex according to claim 1, wherein the pyridylphosphine compound is a ligand.

3. The metal complex according to claim 1 or 2, wherein R², R⁴, R⁵ and R⁷ independently each represent
an alkyl group having 1 to 20 carbon atom and optionally having a halogen atom as a substituent,
an aryl group having 6 to 20 carbon atom and optionally having a halogen atom as a substituent,
a silyl group represented by the following formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent.

4. The metal complex according to claim 3, wherein R¹ is a hydrogen atom, a halogen atom, a linear alkyl group having 1 to 4 carbon atoms, a benzyl group, a phenyl group, a methoxy group, a benzyloxy group or a phenoxy group, R², R⁴, R⁵ and R⁷ independently each is an alkyl group having 1 to 4 carbon atoms, an alkoxy group having 1 to 4 carbon atoms, a benzyl group, a phenyl group, a trimethylsilyl group, a tert-butyldimethylsilyl group or a phenoxy group, and R³ and R⁶ independently each is a hydrogen atom or an alkoxy group having 1 to 4 carbon atoms.

5. The metal complex according to claim 3, wherein R¹ is a hydrogen atom or a linear alkyl group having 1 to 4 carbon atoms, R², R⁴, R⁵ and R⁷ independently each is an alkyl group having 1 to 4 carbon atoms, an alkoxy group having 1 to 4 carbon atoms, a benzyl group, a phenyl group or a phenoxy group, and R³ and R⁶ independently each is a hydrogen atom or an alkoxy group having 1 to 4 carbon atoms.

6. The metal complex according to claim 3, wherein R¹ is a linear alkyl group having 1 to 4 carbon atoms, R², R⁴, R⁵ and R⁷ independently each is an alkyl group having 1 to 4 carbon atoms, and R³ and R⁶ independently each is a hydrogen atom, an alkyl group having 1 to 4 carbon atoms, or an alkoxy group having 1 to 4 carbon atoms.

7. The metal complex according to claim 3, wherein R¹ is a linear alkyl group having 1 to 4 carbon atoms, and R², R⁴, R⁵ and R⁷ are methyl groups.

8. The metal complex according to claim 3, wherein the pyridylphosphine compound is bis(3,5-dimethylphenyl)(6-methyl-2-pyridyl)phosphine, bis(3,5-dimethyl-4-methoxyphenyl)(6-methyl-2-pyridyl)phosphine, or bis(3,5-dimethylphenyl) (6-ethyl-2-pyridyl)phosphine.

9. The metal complex according to claim 3, wherein the pyridylphosphine compound is bis(3,5-dimethylphenyl) (6-methyl-2-pyridyl)phosphine.

10. The metal complex according to claim 3, wherein the pyridylphosphine compound is bis(3,5-dimethyl-4-methoxyphenyl) (6-methyl-2-pyridyl)phosphine.

11. The metal complex according to claim 3, wherein the pyridylphosphine compound is bis(3,5-dimethylphenyl)(6-ethyl-2-pyridyl)phosphine.

12. The metal complex according to claim 3, wherein the metal atom belonging to Group 10 of the periodic table is a palladium atom.

13. A pyridylphosphine compound represented by the formula (1) 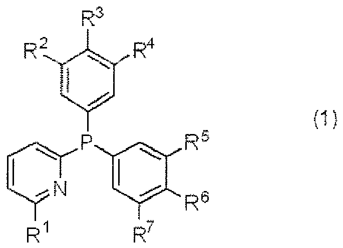 wherein R¹ represents a hydrogen atom, a halogen atom,
a linear alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent,
R², R⁴, R⁵ and R⁷ independently each represent
an alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
a silyl group represented by the following formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent,
R³ and R⁶ independently each represent a hydrogen atom, a halogen atom,
an alkyl group having 1 to 20 carbon atom and optionally having a halogen atom as a substituent,
an aralkyl group having 7 to 20 carbon atom and optionally having a halogen atom as a substituent,
an aryl group having 6 to 20 carbon atom and optionally having a halogen atom as a substituent,
a silyl group represented by the following formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent, and
two groups bonded to the neighboring carbon atoms among R², R³, R⁴, R⁵, R⁶ and R⁷ may be bonded each other to form a ring together with the carbon atoms to which they are bonded, and the formula (2) is represented by 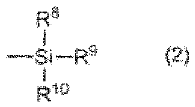 wherein R⁸, R⁹. and R¹⁰ independently each represent a hydrocarbon group having 1 to 20 carbon atoms.

14. The pyridylphosphine compound according to claim 13, wherein R², R⁴, R⁵ and R⁷ independently each represent
an alkyl group having 1 to 20 carbon atom and optionally having a halogen atom as a substituent,
an aryl group having 6 to 20 carbon atom and optionally having a halogen atom as a substituent,
a silyl group represented by the formula (2),
an alkoxy group having 1 to 20 carbon atom and optionally having a halogen atom as a substituent,
an aralkyloxy group having 7 to 20 carbon atom and optionally having a halogen atom as a substituent or
an aryloxy group having 6 to 20 carbon atom and optionally having a halogen atom as a substituent.

15. The pyridylphosphine compound according to claim 13 or 14, wherein R¹ is a hydrogen atom, a halogen atom, a linear alkyl group having 1 to 4 carbon atoms, a benzyl group, a phenyl group, a methoxy group, a benzyloxy group or a phenoxy group, R², R⁴, R⁵ and R⁷ independently each is an alkyl group having 1 to 4 carbon atoms, an alkoxy group having 1 to 4 carbon atoms, a benzyl group, a phenyl group, a trimethylsilyl group, a tert-butyldimethylsilyl group or a phenoxy group, and R³ and R⁵ independently each is a hydrogen atom or an alkyl group having 1 to 4 carbon atoms.

16. The pyridylphosphine compound according to claim 13 or 14, wherein R¹ is a hydrogen atom or a linear alkyl group having 1 to 4 carbon atoms, R², R⁴, R⁵ and R⁷ independently each is an alkyl group having 1 to 4 carbon atoms, an alkoxy group having 1 to 4 carbon atoms, a benzyl group, a phenyl group or a phenoxy group, and R³ and R⁶ independently each is a hydrogen atom or an alkyl group having 1 to 4 carbon atoms.

17. The pyridylphosphine compound according to claim 13 or 14, wherein R¹ is a linear alkyl group having 1 to 4 carbon atoms, R², R⁴, R⁵ and R⁷ independently each is an alkyl group having 1 to 4 carbon atoms, and R³ and R⁶ independently each is a hydrogen atom, an alkyl group having 1 to 4 carbon atoms, or an alkoxy group having 1 to 4 carbon atoms.

18. The pyridylphosphine compound according to claim 13 or 14, which is bis(3,5-dimethylphenyl)(6-methyl-2-pyridyl)phosphine, bis(3,5-dimethyl-4-methoxyphenyl) (6-methyl-2-pyridyl)phosphine, or bis(3,5-dimethylphenyl)(6-ethyl-2-pyridyl)phosphine.

19. A method for producing the pyridylphosphine compound according to claim 13 or 14, which comprises a first step of reacting a halogen-substituted pyridine compound represented by the formula (3) 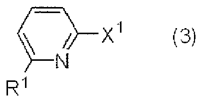 wherein R¹ is the same meaning as defined in claim 13, and X¹ represents a halogen atom, with an alkyllithium compound, and
a second step of reacting the reaction mixture obtained in the first step with a phosphine halide represented by the formula (4) 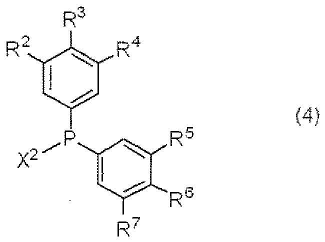 wherein R², R³, R⁴, R⁵, R⁶ and R⁷ are the same meaning as defined in claim 13, and X² represents a halogen atom.

20. A metal complex obtained by contacting the pyridylphosphine compound according to claim 13 or 14 with a compound having a metal atom belonging to Group 10 of the periodic table.

21. A method for producing an alkyl methacrylate comprising a step of reacting an acetylene compound, carbon monoxide and an alcohol in the presence of the metal complex according to claim 3 and a protic acid.
